# EUROPÄISCHE PATENTSCHRIFT

(11) **EP 1 899 983 B1**
(45) Veröffentlichungstag und Bekanntmachung des Hinweises auf die Patenterteilung: **06.04.2016**
(21) Anmeldenummer: 06753569.0
(22) Anmeldetag: 11.05.2006
(51) Int. Cl.: B01L 3/00, G21G 1/00, G21G 4/08

(54) **VORRICHTUNG UND VERFAHREN ZUR HERSTELLUNG EINER GERINGEN MENGE EINER RADIOAKTIVEN SUBSTANZVERBINDUNG**
DEVICE AND METHOD FOR PREPARING A SMALL QUANTITY OF A RADIOACTIVE SUBSTANCE COMPOUND
DISPOSITIF ET PROCEDE POUR PREPARER UNE PETITE QUANTITE D'UN COMPOSE DE SUBSTANCES RADIOACTIVES

(30) Priorität: 07.07.2005 DE 102005031920
(43) Veröffentlichungstag der Anmeldung: 19.03.2008
(73) Patentinhaber: ITM Isotopen Technologien München AG, 85748 Garching (DE)
(72) Erfinder: SONNENHOL, Julian, Bernd, 80797 München (DE); EURSCH, Andreas, 82467 Garmisch Partenkirchen (DE); HARFENSTELLER, Mark, 85716 Unterschleißheim (DE); SCHILP, Michael, 93047 Regensburg (DE); BUCK, Oliver, 83457 Bayerisch Gmain (DE); EHRENFRIED, Lisa, Maria, 74235 Erlenbach (DE); NIKULA, Tuomo, 85521 Ottobrunn (DE)
(74) Vertreter: Appelt, Christian W.
(86) Internationale Anmeldenummer: PCT/EP2006/004438
(87) Internationale Veröffentlichungsnummer: WO 2007/006359

(56) Entgegenhaltungen:
- WO-A-98/22625
- WO-A2-00/79285
- WO-A2-2004/093652
- US-A- 4 853 546
- US-A- 4 853 546
- US-A- 5 674 742

## Beschreibung

Die vorliegende Erfindung betrifft eine Vorrichtung zur Herstellung geringer Mengen einer radioaktiven Substanzverbindung und insbesondere geringer Mengen radiomarkierter Biomoleküle sowie ein entsprechendes Herstellungsverfahren.

In Kliniken werden radioaktive Isotope sowohl bei therapeutischen als auch diagnostischen Anwendungen eingesetzt.

Die von den Isotopen ausgehende radioaktive Strahlung wird für die Behandlung von Krebs, zur Schmerztherapie sowie zur Wundbehandlung verwendet. Hierzu werden radioaktive Isotope auf unterschiedliche Weise in den Körper eingebracht. Möglichkeiten bestehen im Binden an organische stoffwechselnde Agenzien, wie beispielsweise Zucker oder Antikörper, Einspritzen in Körperöffnungen, Einbringen in einer umschlossenen nicht-organischen Hülle, wie einer Nadel, Kapsel oder einem Katheter. Es werden dabei Isotope mit geeigneter Halbwertszeit und Strahlungsart und insbesondere möglichst kurzen Strahlungsreichweiten verwendet.

Bei diagnostischen Anwendungen ermöglichen radioaktive Isotope beispielsweise in bildgebenden Verfahren das Sichtbarmachen von Stoffwechselvorgängen und das spezifische Lokalisieren einzelner Zellarten. Hierzu werden die Isotope in Moleküle eingebaut, die am Stoffwechsel teilnehmen, oder an geeignete Proteine wie monoklonale Antikörper gekoppelt. Für eine möglichst geringe Belastung des Patienten werden kleine Dosisraten sowie eine sehr kurze Halbwertszeit von wenigen Tagen, Stunden bzw. Minuten angestrebt.

Für die Herstellung von mit radioaktiven Isotopen markierten Medikamenten müssen chemisch-physikalische Prozesse unter kontrollierten Bedingungen durchgeführt werden. Aufgrund der Zerfallseigenschaften der Isotope ist eine Herstellung derartiger Medikamente in der Nähe des Veräbreichungsortes, wie beispielsweise einer Klinik erforderlich. Momentan erfolgt die Herstellung derartiger Medikamente in Kliniken häufig mit steckbaren Schlauchverbindungen und vielen Einzelkomponenten. Ein derartiger Aufbau kann jedoch häufig nur für die Herstellung einer einzigen, für einen Patienten individualisierten Medikamentendosis genutzt werden und muß bei jeder Veränderung des Herstellungsprozesses, wie beispielsweise zur Herstellung eines anderen Medikaments für einen anderen Patienten, angepaßt bzw. umgebaut werden. Dazu sind manuelle Eingriffe erforderlich, die mit einer radioaktiven Belastung des Bedienpersonals verbunden sein können. Auch bei der Handhabung von Substanzen während des Herstellungsprozesses kann es zu einer Kontaminierung von Personen kommen.

Des weiteren ist vor bzw. nach jeder Herstellung eines Medikaments eine Sterilisierung und Reinigung zahlreicher Einzelkomponenten, wie beispielsweise von Schläuchen und gefäßen notwendig, wodurch sich der Aufwand zur Herstellung der Medikamente erhöht. Auch kann die Entsorgung einiger Einzelkomponenten erforderlich sein, wodurch die Kosten ebenfalls erhöht werden.

In vielen Fällen werden lediglich geringste Mengen von weniger als 10 ml eines bestimmten radioaktiven Isotope beinhaltenden Medikaments und dementsprechend geringe Mengen an Ausgangssubstanzen benötigt. Die manuelle Handhabung sowie die präzise Dosierung derartiger sehr geringer Mengen gestaltet sich häufig schwierig. Daher werden in der Regel größere, besser handhabbare Mengen eines Medikaments hergestellt, die die zur Verabreichung an einen Patienten benötigte Menge übersteigen. Aufgrund der individualisierten Herstellung kann jedoch der nicht verabreichte Rest in den meisten Fällen nicht weiter verwendet werden und muß entsorgt werden, was ebenfalls zu einer Erhöhung der Kosten führt.

Die momentan praktizierte manuelle Herstellung ist auch aufgrund von Ungenauigkeiten bei der Abmessung insbesondere von kleinen Mengen von Substanzen nachteilig.

WO2004/093652A2 offenbart einen Mikroreaktor mit mehreren Mikrokanälen, die eine Handhabung extrem geringer Volumina von Flüssigkeiten in der Größenordnung von fL bis µL ermöglichen.

US 4, 853, 546 offenbart eine automatische Abfüllvorrichtung für Radioisotope.

WO 98/22625 A1 offenbart eine Mikrovorrichtung zur isothermalen Vervielfältigung von Nukleinsäuren.

WO00/79285 A2 offenbart eine Vorrichtung zur Durchführung mikroanalytischer und mikrosynthetischer Analysen und Verfahren. Offenbart wird eine Mikrosystemplattform und eine Mikromanipulationsvorrichtung zur Manipulation der Plattform, welche die Zentripetalkraft einsetzt, die aus der Drehung der Plattform resultiert, um eine Bewegung von Fluiden durch Mikrokanäle hervorzurufen.

Es ist daher die Aufgabe der vorliegenden Erfindung eine Vorrichtung und eine entsprechendes Verfahren bereitzustellen, mit der bzw. dem eine weitestgehend automatisierte und Patienten-individualisierte Zubereitung einer geringen bedarfsgemäßen Menge einer radioaktiven Substanzverbindung und insbesondere mit Isotopen radiomarkierter Biomoleküle möglich ist.

Diese Aufgabe wird gelöst durch eine Vorrichtung gemäß Anspruch 1 sowie ein entsprechendes Verfahren gemäß Anspruch 19.

Bevorzugte Ausführungsformen der Erfindung sind in den Unteransprüchen angegeben.

Erfindungsgemäß wird eine Vorrichtung zur Herstellung einer geringen Menge einer radioaktiven Substanzverbindung bereitgestellt, die einen einteiligen Körper, eine in den Körper integrierte und zur Aufnahme einer geringen Menge chemischer Substanzen eingerichtete Mischvorrichtung und zumindest ein in den Körper integriertes und mit der Mischvorrichtung verbundenes und zur Aufnahme einer geringen Menge einer chemischen Substanz eingerichtetes Behältnis umfaßt.

Bei der erfindungsgemäßen Vorrichtung handelt es sich im wesentlichen um eine miniaturisierte, kompakte radiopharmazeutische Produktionsanlage, die im wesentlichen automatisiert und daher ohne oder nahezu ohne Eingriff einer Person betrieben werden kann. Mit der Vorrichtung können kleine Volumina einer radiochemischen Substanzverbindung mit präzisen Mischungsverhältnissen im wesentlichen automatisiert hergestellt werden. Aufgrund der integrierten Bauweise, die zumindest ein zur Aufnahme eine kleinen Substanzmenge eingerichtetes Behältnis und eine entsprechend dimensionierte damit verbundene Mischvorrichtung umfaßt, ist eine manuelle Verbindung von Einzelkomponenten nicht notwendig. Die Vorrichtung ist vorzugsweise als eine Einwegkomponente ausgebildet, so daß auch eine Reinigung nicht erforderlich ist, wodurch Zeit, Reinigungsmittel und damit Kosten gespart werden können. Aufgrund der miniaturisierten, an individualisierte Einzeldosen angepaßten Bauweise und Größe der Vorrichtung wird die zur Herstellung verwendete Menge an chemischen Substanzen optimal ausgenutzt, entstehen sehr geringe Verluste von Substanzen z.B. durch Benetzung der Wände der Vorrichtung, können Totvolumina im wesentlichen vermieden werden und kann eine in der Vorrichtung verbleibende Restmenge an Ausgangssubstanzen und hergestellten radioaktiven Substanzverbindungen minimiert werden. Die Vorrichtungen sind kostengünstig mit großen Stückzahlen herstellbar, wobei der innere Aufbau der Vorrichtung je nach Verwendungszweck variiert werden kann.

Die Vorrichtung ist für ein Vermischen von nicht radioaktiven Substanzen, wie z.B. von Biomolekülen, mit radioaktiven Isotopen eingerichtet, wobei die radioaktiven Isotope in der Nähe eines entsprechenden Reaktors bzw. einer Isotopenquelle, die beispielsweise in einer Klinik aufgestellt ist, vorzugsweise über einen verschließbaren Zugang, wie z.B. eine Membran oder eine mechanische Schleuse in die Vorrichtung und insbesondere in die in diese integrierte Mischvorrichtung zugeführt werden. Die nicht radioaktiven Substanzen, wie beispielsweise Biomoleküle oder Pufferlösungen können sich bereits in einem oder mehreren in die Vorrichtung integrierten Behältnissen befinden, oder auch zusammen mit einer oder mehreren radioaktiven Substanzen in die Vorrichtung zugeführt werden.

Die Vorrichtung kann ein oder mehrere in die Vorrichtung integrierte und ein Volumen von weniger als 1 ml, bevorzugt von weniger als 100 µl und besonders bevorzugt weniger als 10 µl fassende Behältnisse aufweisen. Dadurch können die für eine Herstellung einer individualisierten Medikamentenmenge benötigten Ausgangssubstanzen, die über einen ausreichenden Zeitraum lagerfähig sind, in der benötigten Menge bereits in der Vorrichtung gelagert werden. Die Verbindung der chemischen Substanzen mit einer oder mehreren radioaktiven Substanzen findet innerhalb der Vorrichtung und insbesondere in der Mischvorrichtung statt. Da sich kleinste Mengen bei genauer Dosierung innerhalb kürzest möglicher Zeit, wie z.B. innerhalb einiger Millisekunden, nahezu perfekt vermischen lassen, kann gegenüber systemen, die zur Mischung größerer Mengen an Substanzen ausgelegt sind, eine Verbesserung des Bindungsgrads, der Ausbeute und eine Reduktion der Prozeßzeit erreicht werden.

Die Vorrichtung kann insbesondere einen oder mehrere in die Vorrichtung integrierte und ein Volumen von weniger als 5 ml, bevorzugt von weniger als 100 µl und besonders bevorzugt weniger als 10 µl umfassende Kanäle aufweisen. Die Vorrichtung umfaßt somit vorzugsweise für kleinste Mengen chemischer Substanzen geeignete Kanäle, wodurch kurze Verbindungen sichergestellt werden können, die ein rasches und im wesentlichen verlustfreies Vermischen der Substanzen zulassen. Aufgrund ihrer vorzugsweise geringen Abmessungen ist auch zur Herstellung der Vorrichtung insgesamt lediglich eine geringe Materialmenge erforderlich, wodurch die Herstellungskosten gesenkt werden können.

Vorzugsweise weisen die Kanäle eine Höhe von weniger als 500 µm, bevorzugt von weniger als 100 µm und besonders bevorzugt von weniger als 25 µm und eine Breite von weniger als 5 mm, bevorzugt von weniger als 500 µm und besonders bevorzugt von weniger als 100 µm auf. Aufgrund der geringen Querschnittsfläche der Kanäle sind in Verbindung mit Flüssigkeiten auftretende Phänomene, wie z.B. Kapillareffekte, Diffusionseffekte, Brown'sche Molekularbewegung usw. besonders ausgeprägt und können für Förder- und Mischmechanismen ausgenutzt werden.

Die Kanäle dienen in erster Linie der Verbindung zwischen der Mischvorrichtung und einem oder mehreren in der Vorrichtung vorgesehenen Behältnissen. Sie können jedoch auch zur Verbindung zwischen einzelnen Behältnissen dienen. Gemäß einer speziellen Ausführungsform kann die Mischvorrichtung selbst durch einen Kanal gebildet sein. Die Vermischung erfolgt dann durch Zuführen zweier zu vermischender flüssiger Substanzen jeweils von einer Seite des Kanals, wobei sich die Substanzen bei ihrem Aufeinandertreffen miteinander vermischen und der Mischvorgang durch die oben genannten mikrofluidischen Effekte unterstützt und beschleunigt wird.

Gemäß einer bevorzugten Ausführungsform wird die Mischvorrichtung aus der Gruppe umfassend Kaskadenmischer, Diffusionsmischer, Laminationsmischer, auf dem SplitRecombine-Prinzip arbeitende Mischer, mit Hilfe von elektrischen Wechselfeldern oder mit Schall- bzw. Schwingungsunterstützung arbeitende Mischer gewählt. Diese Mischer sind insbesondere zur Vermischung kleinster Mengen von Substanzen geeignet und arbeiten unter Ausnutzung von bei kleinen Flüssigkeitsmengen signifikant auftretenden und dem Fachmann bekannten physikalischen Phänomenen, wie z.B. dem Kapillareffekt, der Brown'schen Molekularbewegung usw.

Insbesondere weist die Mischvorrichtung eine Aufnahmekapazität von weniger als 1 ml, bevorzugt von weniger als 100 µl und besonders bevorzugt von weniger als 10 µl auf. Die hergestellte radiochemische Substanzverbindung kann somit mit einer an eine einzige Verabreichungsdosis angepaßten Menge produziert werden, so daß die Notwendigkeit einer Entsorgung von Überschußmengen vermieden werden kann.

Ferner ist die Vorrichtung gemäß einer bevorzugten Ausführungsform nach außen abgeschlossen bzw. abgedichtet. Damit kann ein Austritt von chemischen Substanzen und eine Kontaminierung von Personen vermieden werden. Auch eine Kontaminierung der in der Vorrichtung gelagerten Substanzen kann ausgeschlossen werden. Die Abdichtung nach außen wird in erster Linie bereits durch die integrierte Bauweise der Vorrichtung erzielt. Darüber hinaus können verschließbare oder abdichtbare Zugänge, wie z.B. Membranen oder mechanische Schleusen vorgesehen werden.

Gemäß einer weiteren Ausführungsform umfaßt die Vorrichtung zumindest einen Zugang, insbesondere für einen Sensor, und/oder eine mechanische Schnittstelle zur Außenseite. Mit Hilfe des Zugangs können Sensor- und Meßvorrichtungen zur Durchführung einer Qualitätskontrolle ausgeführt werden. Über eine oder mehrere mechanische Schnittstellen, wie beispielsweise Membranen oder Schleusen, können auch chemische Substanzen und insbesondere radioaktive Isotope von außen in die Mischvorrichtung zugeführt werden.

Gemäß einer weiteren Ausführungsform umfaßt die Vorrichtung zumindest ein Förder- bzw. Dosiermittel, aus der Gruppe umfassend, unter Einsatz der Zentrifugalkraft, eine elektrische auf ein Fluid wirkende Kraft, eine Druck- oder Volumenänderung oder eines schall- oder schwingungsunterstützten Förderverfahrens arbeitende Mittel oder einen Teil davon. Durch den Einsatz von Förder- bzw. Dosiermitteln mit einer entsprechenden Dosierpräzision können exakte Mengen einer chemischen Substanz in die Mischvorrichtung zugeführt werden.

Ferner kann die Vorrichtung zumindest ein Meß- oder Sensormittel, insbesondere zur Erfassung einer physikalischen Größe aus der Gruppe umfassend die Art und Stärke radioaktiver Strahlung, den pH-Wert, die Temperatur, ein Mittel zur Durchführung einer Chromatographie oder Elektrophorese und/oder ein Mittel zur Erfassung der Lichtbrechung und/oder Erfassung zumindest einer Eigenschaft eines Stoffes aus der Gruppe umfassend das Vorhandensein oder die Abwesenheit, die Menge, die Farbe und den Brechungsindex, eine Ionenaustauschsäule, eine Größenausschlußextraktionssäule oder einen Teil davon umfassen. Durch das Vorsehen eines oder mehrerer dieser Mittel kann eine Qualitätssicherung der hergestellten radioaktiven Substanzverbindung gewährleistet werden und können auch die Ausgangssubstanzen, die in die Vorrichtung zugeführt werden oder sich bereits in dieser befinden anhand geeigneter chemischer oder physikalischer Parameter auf ihre Qualität und bezüglich der vorhandenen Menge überprüft werden. Die Meß- oder Sensormittel sind vorzugsweise aus Kostengründen lediglich teilweise in der Vorrichtung vorgesehen bzw. in diese integriert, während sich in Verbindung mit einer Vielzahl von Vorrichtungen immer wieder verwendbare elektronische und mechanische Komponenten dieser Mittel vorzugsweise außerhalb der Vorrichtung befinden. Durch die Meß- oder Sensormittel kann der Grad der Automatisierung des Herstellungsprozesses zusätzlich erhöht werden.

Zur Automatisierung der Herstellungsprozesse ist die Vorrichtung gemäß einer weiteren Ausführungsform vorzugsweise extern ansteuerbar und umfaßt Mittel zur Identifikation des Vorrichtungstyps sowie Mittel zum Empfang von Steuerungs- und/oder Stromversorgungssignalen für gegebenenfalls vorgesehene Förder- bzw. Dosiermittel, Meß- oder Sensormittel, Mittel zur Durchführung einer Chromatographie oder Elektrophorese, Mittel zur Erfassung der Lichtbrechung, zur Erfassung von Eigenschaften eines Stoffs, wie z.B. sein Vorhandensein, die Menge, die Farbe, Brechungsindex usw., für eine Ionenaustauschsäule oder eine Größenausschlußextraktionssäule. Die Mittel zum Empfang von Steuerungs- und/oder Stromversorgungssignalen können beispielsweise durch unmittelbar auf der Vorrichtung vorgesehene und insbesondere durch Verdampfungs- oder Sputtertechniken hergestellte Leitungen oder durch Transceivereinrichtungen, induktive Energieübertragungseinrichtungen, auf Basis von IR oder RF-Signalen arbeitende Femsteuerungseinrichtungen usw. gebildet werden.

Gemäß einer weiteren bevorzugten Ausführungsform sind zumindest Teile der Vorrichtung monolithisch und insbesondere mittels Mikroverfahrenstechnik, umfassend Mikrospritzgieß- oder Mikroprägetechnik, hergestellt. Dadurch kann eine kostengünstige Herstellung von Teilen der Vorrichtung mit hohen Stückzahlen erfolgen. Zur Fertigstellung der einzelnen Vorrichtung können die mikroverfahrenstechnisch hergestellten Teile mittels herkömmlicher Techniken, wie z.B. durch Verschweißen oder Kleben verbunden werden. Die Mikroverfahrenstechnik eignet sich gut zur präzisen Ausbildung der kleinen Kanäle, Behältnisse und Mischvorrichtungen bzw. entsprechender Aufnahmebereiche für diese in der Vorrichtung. Die monolithische Bauweise trägt des weiteren inhärent zur Abdichtung der Vorrichtung nach außen bei, da alle Elemente der Vorrichtung im Inneren vorgesehen bzw. ausgebildet werden können und die Vorrichtung nach ihrer Fertigstellung bis auf verschließbare Zugänge nach außen abgeschlossen ist, so daß ein Austritt von Substanzen und damit die Gefahr einer Kontaminierung von Personen vermieden werden kann.

Vorzugsweise umfaßt die Vorrichtung im Inneren beschichtete Flächen zur Verhinderung des Anhaftens von Stoffen oder zur Katalyse von chemischen Reaktionen. Zur Bescheunigung bzw. Beeinflussung der Mischvorgänge oder Reaktionen kann die Vorrichtung auch eine Heiz- und/oder Kühlvorrichtung oder zumindest einen Teil davon, wie z.B. einen Heizdraht oder ein Peltier-Element umfassen. Vorzugsweise ist die Vorrichtung aus Kunststoff, insbesondere aus Polyethylen, Polypropylen, PMMA, PC, PTFE, Cyclo-Olefin-Copolymer, Silizium, Metall oder Glas oder einer Kombination daraus hergestellt.

Die Vorrichtung kann ferner in ein System integriert sein, das eine mit der Vorrichtung koppelbare Steuerungseinheit umfaßt. Damit kann ein hoher Automatisierungsgrad bei der Herstellung von Patienten-individualisierten Medikamentendosen erreicht werden. Die Herstellungsprozesse sind reproduzierbar und können auch einfach individualisiert bzw. variiert werden, wobei jede Vorrichtung vorzugsweise lediglich einmal verwendet wird und dazu mit einer immer wieder verwendbaren Steuerungseinheit gekoppelt werden kann.

Das System umfaßt des weiteren vorzugsweise eine mit der Vorrichtung koppelbare Isotopenquelle.

Schließlich kann das System ein mit der Vorrichtung koppelbares Förder- bzw. Dosiermittel, Meß- oder Sensormittel, ein Mittel zur Durchführung einer Chromatographie oder Elektrophorese und/oder ein Mittel zur Erfassung der Lichtbrechung und/oder Erfassung zumindest einer Eigenschaft eines Stoffes aus der Gruppe umfassend das Vorhandensein oder die Abwesenheit die Menge, die Farbe und den Brechungsindex, eine Ionenaustauschsäule, eine Größenausschlußextraktionssäule oder einen Teil davon, ein Sensorgerät für Radioaktivität, wie z.B. einen Szintillationszähler, oder einen Teil davon umfassen.

Durch das Vorsehen dieser Mittel außerhalb der Vorrichtung können Kosten reduziert werden, da diese Einrichtungen, die vorzugsweise zur Qualitätssicherung verwendet werden, mit einer Vielzahl von Vorrichtungen zur Herstellung individueller Medikamentendosen verwendet werden können.

Erfindungsgemäß wird ferner ein Verfahren zur Herstellung einer geringen Menge einer radioaktive Substanzverbindung vorgesehen. Es umfaßt die Schritte
- eines Bereitstellens einer zur Herstellung einer geringen Menge einer chemischen Substanzverbindung eingerichteten Vorrichtung, die einen einteiligen Körper, eine in den Körper integrierte und zur Aufnahme einer geringen Menge chemischer Substanzen eingerichtete Mischvorrichtung und zumindest ein in den Körper integriertes und mit der Mischvorrichtung verbundenes und zur Aufnahme einer geringen Menge einer chemischen Substanz eingerichtetes Behältnis umfaßt,
- das Zuführen einer geringen Menge zumindest einer Substanz in eine Mischvorrichtung der Vorrichtung,
- das Zuführen einer geringen Menge zumindest einer radioaktiven Substanz in die Mischvorrichtung,
- das Mischen der zumindest einen Substanz mit der zumindest einen radioaktiven Substanz und
- das Entnehmen der hergestellten Substanzverbindung.

Mit dem erfindungsgemäßen Verfahren können geringe für eine einzige individuelle Verabreichung an einen Patienten benötigte Mengen radioaktiver Substanzverbindungen und insbesondere mit Isotopen radiomarkierte Biomoleküle hergestellt werden. Da mittels des erfindungsgemäßen Verfahrens geringste Mengen radioaktiver Substanzverbindungen hergestellt werden können, entstehen im Gegensatz zu herkömmlichen manuellen Zubereitungsmethoden keine überschüssigen Mengen an Substanzverbindungen, die dann entsorgt werden müssen. Das erfindungsgemäße Verfahren kann weitestgehend automatisiert durchgeführt werden, wobei eine Kontaminierung von Personen im wesentlichen ausgeschlossen ist. Ferner kann auf Reinigungs- und Sterilisierungsmaßnahmen im wesentlichen verzichtet werden, da die im Verfahren verwendete Mikrofluid-Vorrichtung vorzugsweise als Einwegkomponente ausgelegt ist und nach einem einmaligen Gebrauch entsorgt werden kann. Aufgrund der serienmäßigen Herstellbarkeit der Mikrofluid-Vorrichtung, sind die Herstellungsprozesse von Substanzverbindungen reproduzierbar. Durch Abändern des Aufbaus der Mikrofluid-Vorrichtung können unterschiedliche Zubereitungsprozesse durchgeführt werden. Individualisierte Medikamente können jedoch auch mit demselben Aufbau bzw. Typ der Vorrichtung, jedoch unterschiedlichen Ausgangssubstanzen und Mengen erzielt werden.

Das Zuführen einer geringen Menge einer Substanz umfaßt gemäß einer Ausführungsform insbesondere das Zuführen einer Menge von weniger als 2 ml, bevorzugt von weniger als 1 ml und besonders bevorzugt von weniger als 100 µl. Derartig geringe Mengen werden z.B. zur Vorbereitung von Einzeldosen radiomarkierter Biomoleküle benötigt.

Bevorzugter Weise umfaßt das Zuführen des weiteren das Zuführen einer Substanz aus einem in die Vorrichtung integrierten Behältnis. In einem derartigen Behältnis werden ausschließlich über einen längeren Zeitraum lagerfähige Chemikalien aufbewahrt, die für den Herstellungsprozeß benötigt werden. Vorzugsweise wird zumindest eine Substanz bereits bei der Herstellung der Vorrichtung in ein Behältnis der Vorrichtung eingebracht. Dies kann beim Hersteller der Vorrichtung oder bei einem Pharmahersteller erfolgen. Nach dem Einbringen der Substanz ist eine Kontaminierung von bzw. durch Personen, die mit der Vorrichtung in Kontakt kommen, weitestgehend ausgeschlossen.

Ferner erfolgt das Zuführen einer radioaktiven Substanz vorzugsweise von außerhalb der Vorrichtung, wie beispielsweise aus einem Isotopengenerator oder einem geeigneten Behälter. Somit kann das Zuführen der radioaktiven Substanz zur Herstellung der radiochemischen Substanzverbindung und insbesondere zur Radiomarkierung von Biomolekülen unmittelbar am Verabreichungsort, d.h. in einer Klinik, erfolgen. Ferner kann das Verfahren gemäß einer Ausführungsform auch das Kühlen oder Erwärmen von Substanzen umfassen.

Gemäß einer weiteren Ausführungsform umfaßt das Verfahren das Durchführen einer Qualitätskontrolle einer oder mehrerer Substanzen oder Substanzverbindungen in der Vorrichtung. Schließlich kann gemäß einer weiteren Ausführungsform auch die hergestellte radiochemische Substanzverbindung vor einer Entnahme einer Qualitätskontrolle unterzogen werden. Die Qualitätskontrolle kann das Durchführen einer Größenausschlußchromatographie, einer Ionenaustauschchromatographie und/oder einer Dünnschichtchromatographie umfassen.

Vorzugsweise umfaßt das Mischen das Radiomarkieren von Biomolekülen, wie beispielsweise Antikörpern und Peptiden, mit Isotopen. Dabei kann das Mischen eine chemische Bindung zwischen der radioaktiven Substanz und der zumindest einen weiteren Substanz zur Folge haben. Die radioaktive Substanz kann vorzugsweise aus der Gruppe umfassend Me²⁺, Me³⁺ und MeO₄⁻, Halogene, Cobalt-57, Cobalt-58, Selenium-75, Gallium-67, Gallium-68, Iod-123, Iod-124, Iod-125, Iod-131, Astatin-211, Actinium-225, Bismuth-212, Bismuth-213, Blei-212, Technetium-99m, Rhenium-186, Rhenium-188, Silber-111, Indium-111, Platinum-197, Palladium-109, Kupfer-67, Phosphor-32, Phosphor-33, Yttrium-90, Scandium-47, Samarium-153, Ytterbium-169, Lutetium-177, Rhodium-105, Praseodymium-142, Praseodymium-143, Terbium-161, Holmium-166, Thallium-201 oder Gold-199 gewählt sein.

Ferner kann das Verfahren das Zuführen einer Pufferlösung, die gewählt ist aus der Gruppe umfassend Acetat und Citrat, MES, HEPES, Phosphanate, Carbonate sowie deren Mischungen, oder einer anderen geeigneten Pufferlösung umfassen. Schließlich kann das Verfahren das Steuern und Überwachen des Verfahrensablaufs mittels einer mit der Vorrichtung koppelbaren Steuerungseinheit umfassen. Dadurch kann eine weitgehende Automatisierung des Herstellungsprozesses erreicht werden.

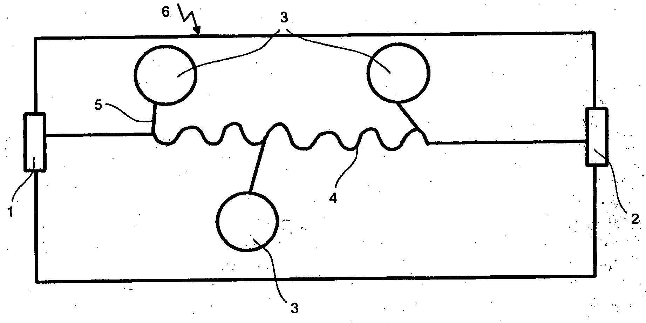

Ein Beispiel einer erfindungsgemäßen Vorrichtung gemäß einer Ausführungsform wird im folgenden anhand der beigefügten Zeichnung erläutert.

Diese zeigt eine schematische Ansicht einer Vorrichtung bzw. Mikrofluid-Vorrichtung zur Herstellung einer geringen Menge einer radioaktiven Substanzverbindung im Querschnitt.

Die Vorrichtung umfaßt einen einteiligen, ggf. aus mehreren Teilen zusammengesetzten Körper, der beispielsweise aus Silizium oder einem Kunststoff hergestellt sein kann. In der Vorrichtung sind drei Behältnisse 3 zur jeweiligen Aufnahme einer geringen Menge einer chemischen Ausgangssubstanz vorgesehen. Die Behältnisse 3 sind in die Vorrichtung integriert und nach außen abgeschlossen. Sie können chemische Substanzen aufnehmen, die gegebenenfalls bereits bei der Herstellung der Vorrichtung durch einen Pharmahersteller in die Vorrichtung eingebracht werden. Je nach Verwendungszweck können alle oder nur einige Behältnisse 3 gefüllt sein. Vorzugsweise handelt es sich bei den in die Vorrichtung eingebrachten Substanzen um lagerbare und zeitlich im wesentlichen unveränderliche Substanzen. Die Behältnisse umfassen ein Volumen in Größenordnung einiger ml. Im wesentlichen in Richtung der Längsachse der Vorrichtung ist ein Kanal 4 vorgesehen, der beispielsweise durch einen Ätzprözeß im Siliziumkörper hergestellt wird. Auch der in der Zeichnung lediglich schematisch gezeigte Kanal 4 umfaßt ein sehr geringes Volumen von einigen ml und weist im mittleren Bereich, der etwa ein Drittel der Länge der Vorrichtung umfaßt, zwischen zwei geraden Abschnitten einen welligen Verlauf auf. Im mittleren Bereich ist ein sogenannter "Snake-Mischer" definiert. Die Vorrichtung weist ferner weitere Kanäle 5 auf, mit welchen die Behältnisse 3 mit dem zentralen Kanal 4 verbunden sind. Auch diese Kanäle sind durch Ätzen hergestellt. Bei der in der Figur gezeigten Ausführungsform dient der zentrale Kanal 4 als Mischvorrichtung für die chemischen Substanzen aus den Behältnissen 3. Die Vorrichtung umfaßt des weiteren Verbindungsteile 1 und 2, wie beispielsweise Membranen oder mechanische Schleusen, am vorderen bzw. hinteren Ende des zentralen Kanals 4, durch die gelöste. Isotope von einer daran angeschlossenen Isotopenquelle (nicht gezeigt) in die Mikrofluid-Vorrichtung zugeführt werden können bzw. aus dieser entnommen werden können.

Die schematisch dargestellte Mikrofluid-Vorrichtung ist lediglich beispielhaft angegeben und es können zahlreiche Modifizierungen daran vorgenommen werden. So können z.B. Zugänge (nicht gezeigt) vorgesehen werden, durch welche Sensor- und Meßmittel u.a. zur Qualitätskontrolle eingeführt werden können. Die Vorrichtung kann des weiteren den Behältnissen zugeordnete Dosier- und Fördermittel oder Teile davon aufweisen, mittels derer die sich in den Reservoirs 3 befindenden chemischen Substanzen mit vorbestimmten Mengen in den zentralen Kanal 4 zugeführt werden können. Im Gegensatz zur dargestellten Ausführungsform wären auch Behältnisse 3 mit unterschiedlichen Volumina denkbar. Auch die Zahl der Kanäle und Behältnisse und deren Anordnung kann zwischen verschiedenen Ausführungsformen variieren und kann von der Zahl der zur Herstellung der Substanzverbindung verwendeten Einzelsubstanzen abhängen. Die Vorrichtung umfaßt vorzugsweise geringe Abmessungen, wie beispielsweise eine Länge von weniger als 10 cm, eine Breite von weniger als 5 cm und eine Höhe von weniger als 2 cm. Jedoch sind die Abmessungen der Vorrichtung variabel und können z.B. durch die Zahl und Abmessungen der Behältnisse, Mischvorrichtungen, Förder- und Dosiermittel usw., d.h. der in die Vorrichtung integrierten Komponenten bestimmt sein.

Ferner kann die Vorrichtung zur Durchführung einer automatisch gesteuerten Herstellung und zur Qualitätsüberwachung Förder- bzw. Dosiermittel, Meß- oder Sensormittel, Mittel zur Durchführung einer Chromatographie oder Elektrophorese, usw. oder Teile dieser und Mittel zum Empfang von Steuerungs- und/oder Stromversorgungssignalen für diese Mittel umfassen.

Die Vorrichtung wird vorzugsweise mit einer entsprechenden Steuereinheit (nicht gezeigt) gekoppelt, die beispielsweise einen programmierbaren Prozessor oder Software enthält. Damit kann unter eine im wesentlichen automatisierte Herstellung von chemischen Substanzverbindungen erfolgen.

Eine fertiggestellte radioaktive Substanzverbindung, wie beispielsweise mit Isotopen radiomarkierte Biomoleküle, können durch ein Verbindungsteil 2 entnommen werden.

Die erfindungsgemäße Vorrichtung kann in einem radiochemischen Produktionssystem für pharmazeutische Produkte zur Krebstherapie oder Diagnose eingesetzt werden. Die Vorrichtung umfaßt dann beispielsweise Reservoirs mit den zur Herstellung eines entsprechenden Medikaments notwendigen Ausgangsstoffen, wie beispielsweise Pufferlösungen, Radikalfänger, monoklonale Antikörper usw.

Mittels der erfindungsgemäßen Vorrichtung und des Verfahrens können beispielsweise monoklonale Antikörper und andere Biomoleküle radiomarkiert werden.

Die Radiomarkierung von Biomolekülen mit Metall-Isotopen (Me²⁺ und Me³⁺) erfordert das Anfügen funktionaler Gruppen an Biomoleküle, wie beispielsweise von Chelaten, wie z.B. EDTA-, DTPA-, DOTA-Derivaten oder anderen Chelaten. Die funktionale Gruppe hat die Aufgabe, eine ausreichend stabile Bindung mit den Metallionen herzustellen. Einige Biomoleküle, wie beispielsweise viele Phosphonatverbindungen, stellen bereits selbst Chelate dar. Während der letzten Jahre wurden auch Peptide entwickelt, die ebenfalls einen Metallionenbindungsteil in ihrer molekularen Struktur aufweisen.

Die Me²⁺ und Me³⁺-Radioisotope werden typischerweise in verdünnter HCl oder HNO₃-Säurelösung bereitgestellt. Die erforderliche Menge radioaktiver Isotope wird in eine Pufferlösung (typischerweise Acetat oder Citrat) eingeführt, um den pH-Wert zu regulieren und in einen Bereich zu bringen, der sich einerseits für die Bindung der Metallionen an die Chelate eignet und andererseits Beschädigungen der Biomoleküle ausschließt.

Die erforderliche Biomolekülmenge wird zur gepufferten Isotopenlösung zugeführt, woraufhin der Mischvorgang bzw. die Reaktion beginnt. Die Reaktionszeit hängt von der Art des Chelats und dem radioaktiven Metall ab. Für einige Prozesse ist eine erhöhte Temperatur erforderlich. Nach der Reaktionszeit werden die Biomoleküle mittels einer Größenausschluß- oder Ionenaustauschchromatographie oder einem anderen geeigneten Verfahren von den ungebundenen radioaktiven Metallionen gereinigt. In einigen Fällen können die radiomarkierten Biomoleküle auch direkt als radiopharmazeutische Substanz mit ausreichenden Mengen and Puffern formuliert werden, ohne daß ein Reinigungsschritt erforderlich ist. Anschließend kann eine Qualitätskontrolle der radiopharmazeutischen Substanzen mit Hilfe geeigneter Verfahren, wie beispielsweise einer Größenausschlußchromatographie, einer Ionenaustauschchromatographie, einer Dünnschichtchromatographie usw. durchgeführt werden.

Ein weiterer beispielhafter Prozeß umfaßt die Radiomarkierung von Biomolekülen mit (Metall)O₄⁻ Isotopen. In der Nuklearmedizin wird häufig TcO₄⁻ verwendet. Das chemische Analogon ReO₄⁻ findet zunehmend bei therapeutischen Anwendungen Interesse. Beide Isotope werden üblicherweise in einer Salzlösung gehalten. Die Radiomarkierung von Biomolekülen mit MeO₄⁻-Isotopen erfordert, daß zunächst das MeO₄⁻-Isotop in einen niedrigeren Oxidationszustand (Me(IV)) oder (Me(V)) reduziert wird, wobei typischerweise Sn²⁺-Ionen als Reduktionsmittel verwendet werden. Das Prinzip des Radiomarkierungsprozesses ist im wesentlichen dasselbe wie das zuvor dargestellte für die Radiomarkierung von Biomolekülen mit Me²⁺ und Me³⁺-Isotopen. Obwohl sowohl Technetium- und Rhenium-Ionen Komplexe mit EDTA-, DTPA-, DOTA-Derivaten bilden, werden üblicherweise für diese Metalle speziell entworfene Chelate in Verbindung mit größeren Biomolekülen verwendet.

Ein anderer beispielhafter Prozeß, der unter Verwendung der erfindungsgemäßen Vorrichtung sowie des erfindungsgemäßen Verfahrens durchgeführt werden kann, ist die Radiomarkierung von Biomolekülen mit Halogenen wie Iod oder Astatin. Diese kann im wesentlichen in zwei Verfahren unterteilt werden. Gemäß einem Verfahren werden Proteine durch Halogenieren einer Tyrosin-Gruppe in der Proteinstruktur radiomarkiert, sofern das Biomolekül einen Tyrosinring (Phenol) oder eine ähnliche Struktur umfaßt. Für die Reaktion wird ein Oxidationsmittel, wie beispielsweise Chloramin-T oder Iodogen, verwendet. Im allgemeinen ist das Verfahren ähnlich zu dem der Radiomarkierung von Biomolekülen mit Me²⁺ und Me³⁺-Isotopen, wobei jedoch in der Reaktionslösung ein Oxidationsmittel vorgesehen wird. Im Ergebnis werden jedoch im Gegensatz zur Markierung mit Me²⁺ und Me³⁺-Isotopen kovalente Bindungen zwischen Iod und Kohlenstoff im Tyrosin-Ring gebildet. Im allgemeinen ist ferner ein Reinigungsprozeß erforderlich. Gemäß einem anderen Verfahren zur Radiomarkierung von Biomolekülen mit Halogenen können Biomoleküle durch Anfügen einer Trialkylzinn-Gruppe, wie beispielsweise einer Trimethylzinn- oder Tributylzinn-Gruppe, an Biomolekül halogeniert werden. Diese können dann unter Oxidationsbedingungen durch ein Halogen substituiert werden.

Zur Durchführung eines beispielhaft̃en Mischvorgangs bzw. Herstellungsprozesses wird die Vorrichtung von entsprechend geschulten Personen aus einer Schutzumhüllurig entnommen und an eine Steuerungseinheit angeschlossen. Ferner werden die sterilen Anschlüsse, z.B. durch Entfernen einer Folie, freigelegt und wird eine Fluid-Verbindung zu einem Isotopenreservoir sowie einem Aufnahmebehälter für das fertige Produkt hergestellt. Es werden ferner die Art und Funktionstüchtigkeit der Vorrichtung überprüft und sichergestellt. Falls erforderlich, können mittels der Steuerungseinheit über an der Mikrofluid-Vorrichtung vorgesehene Anschlüsse elektrische Komponenten, die in die Mikrofluid-Vorrichtung integriert sind, mit Strom versorgt werden. Die Steuerung der in der Mikrofluid-Vorrichtung vorgesehenen Komponenten erfolgt mittels der angeschlossenen Steuerungseinheit.

Beim Start des Produktionsvorgangs durch die Steuerungseinheit werden die einzelnen Substanzen mittels der in die Vorrichtung integrierten oder an dieser vorgesehenen Förder- bzw. Dosiermittel (Pumpen) im richtigen Verhältnis gemäß einer patientenindividuellen Therapieplanung zueinander dosiert und mittels der Mischvorrichtung miteinander vermischt. In einem ersten Reservoir 3 der Vorrichtung befindet sich z.B. ein Acetatpuffer (0.2 M), während in einem weiteren Reservoir eine Menge einer Antikörpersubstanz untergebracht ist. Gegebenenfalls ist in einem weiteren Reservoir eine Menge einer Chelat-Bildner-Substanz angeordnet. Mittels eines durch eine angeschlossene Steuerungseinheit gesteuerten Dosiermittels wird eine gewünschte Menge des Acetatpuffers in die Mischvorrichtung bzw. den zentralen Kanal 4 zugeführt. Des weiteren wird durch die Verbindungseinheit 1 z.B. eine Yttrium-90-Lösung (z.B. 100mCi/ml in 0,04 M HCl) von einer Isotopenquelle in die Mischvorrichtung zugeführt. Darauffolgend wird der pH-Wert der Substanzmischung in der Mischvorrichtung mittels eines geeigneten Meßsensors ermittelt. Falls der pH-Wert unter 5 liegt, werden unter der Steuerung durch die Steuereinheit und mittels einer dem entsprechenden Reservoir zugeordneten Dosiervorrichtung weiterer Acetatpuffer in die Mischvorrichtung zugeführt und gegebenenfalls eine weitere pH-Messung durchgeführt. Schließlich wird aus dem Reservoir eine erforderliche Menge der Antikörpersubstanz in die Mischvorrichtung zugeführt. Danach ruht die Reaktionsmischung für eine ausreichende Zeitdauer. Zusätzlich kann die Reaktionsmischung unter Verwendung einer in die Vorrichtung integrierten oder externen Größenausschlußchromatographievorrichtung gereinigt werden. Nach Ablauf einer entsprechenden Reaktionszeit werden verschiedene Qualitätskontrollprüfungen durchgeführt. Schließlich kann eine radiopharmazeutische Konditionierung (Volumen, Pufferung, isotonisch) erfolgen.

Nach dem Misch- bzw. Reaktionsprozeß wird das zubereitete Medikament in einen Transportbehälter überführt und dann zum Behandlungsort transportiert.

Nach Verabreichung des hergestellten Medikaments wird die Vorrichtung schließlich in einen Äbklingbehälter eingebracht, wobei die Vorrichtung nach dem Abklingen der Radioaktivität ggf. zerlegt wird, die lediglich für eine einmalige Verwendung vorgesehenen Komponenten entsorgt werden und ggf. mehrfach verwendbare Komponenten einer Reinigung unterzogen werden. Durch Einsetzen neuer steriler Einwegbauteile ist die Kassette wieder für eine Befüllung bereitgestellt und kann für eine erneute Herstellung einer Substanzverbindung verwendet werden. Die Befüllung der Vorrichtung kann bei einem Pharma- oder Biotechnologieunternehmen und vorzugsweise unter sterilen und cGMP-gemäßen Verhältnissen erfolgen, so daß die erforderliche Qualität der Substanzen sichergestellt werden kann.

Die in der Beschreibung und in den Ansprüchen offenbarten Merkmale können einzeln oder in beliebigen Kombinationen für die Erfindung von Bedeutung sein.

## Patentansprüche

1. Vorrichtung zur Herstellung einer geringen Menge einer radioaktiven Substanzverbindung, die umfasst:
- einen einteiligen Körper (6),
- eine in den Körper (6) integrierte und zur Aufnahme einer geringen Menge chemischer Substanzen eingerichtete Mischvorrichtung (4), und
- zumindest ein in den Körper (6) integriertes und mit der Mischvorrichtung (4) verbundenes und zur Aufnahme einer geringen Menge einer chemischen Substanz eingerichtetes Behältnis (3), **dadurch gekennzeichnet, dass**
- die Mischvorrichtung (4) einen Kanal mit einer Höhe von weniger als 500µm und eine Breite von weniger als 5mm umfasst, so dass auf eine Flüssigkeit im Kanal ein Kapillareffekt ausgeübt wird, und der Kanal einen welligen Verlauf zwischen zwei geraden Abschnitten aufweist und
- die Vorrichtung eine mechanische Schnittstelle (1, 2) zur Außenseite aufweist und mittels der mechanischen Schnittstelle (1, 2) verschließbar und nach außen abdichtbar ist.

2. Vorrichtung nach Anspruch 1, **dadurch gekennzeichnet, dass** sie ein oder mehrere in die Vorrichtung integrierte und ein Volumen von weniger als 5ml, bevorzugt von weniger als 100µl und besonders bevorzugt von weniger als 10µl umfassende Behältnisse (3) aufweist.

3. Vorrichtung nach Anspruch 1 oder 2, **dadurch gekennzeichnet, dass** sie einen oder mehrere in die Vorrichtung integrierte und ein Volumen von weniger als 1 ml, bevorzugt von weniger als 100µl und besonders bevorzugt von weniger als 10µl umfassende Kanäle (5) aufweist.

4. Vorrichtung nach Anspruch 3, **dadurch gekennzeichnet, dass** der Kanal (5) eine Höhe von weniger als 100µm und bevorzugt von weniger als 25µm und eine Breite von weniger als 500µm und bevorzugt von weniger als 100µm aufweist.

5. Vorrichtung nach einem der Ansprüche 2 bis 4, **dadurch gekennzeichnet, dass** die Mischvorrichtung (4) und das zumindest eine Behältnis (3) durch einen oder mehrere Kanäle (5) verbunden sind.

6. Vorrichtung nach einem der vorhergehenden Ansprüche, **dadurch gekennzeichnet, dass** die Mischvorrichtung (4) eine Aufnahmekapazität von weniger als 1 ml, bevorzugt von weniger als 100µl und besonders bevorzugt von weniger als 10µl aufweist

7. Vorrichtung nach einem der vorhergehenden Ansprüche, **dadurch gekennzeichnet, dass** die Vorrichtung verschließbar und nach außen abdichtbar ist.

8. Vorrichtung nach einem der vorhergehenden Ansprüche, **dadurch gekennzeichnet, dass** sie zumindest einen Zugang, insbesondere für einen Sensor aufweist.

9. Vorrichtung nach einem der vorhergehenden Ansprüche, **dadurch gekennzeichnet, dass** sie zumindest ein Förder- bzw. Dosiermittel aus der Gruppe umfassend unter Einsatz der Zentrifugalkraft, einer elektrischen auf ein Fluid wirkenden Kraft, einer Druck- oder Volumenänderung oder eines schall- oder schwingungsunterstützten Förderverfahrens arbeitende Mittel oder einen Teil davon umfasst.

10. Vorrichtung nach einem der vorhergehenden Ansprüche, **dadurch gekennzeichnet, dass** sie zumindest ein Mess- oder Sensormittel, insbesondere zur Erfassung einer physikalischen Größe aus der Gruppe umfassend die Art und Stärke radioaktiver Strahlung, den pH-Wert, die Temperatur, ein Mittel zur Durchführung einer Chromatographie oder Elektrophorese, und/oder ein Mittel zur Erfassung der Lichtbrechung und/oder Erfassung zumindest einer Eigenschaft eines Stoffes aus der Gruppe umfassend das Vorhandensein oder die Abwesenheit, die Menge, die Farbe und den Brechungsindex, eine Ionenaustauschsäule, eine Größenausschlussextraktionssäule oder einen Teil davon umfasst.

11. Vorrichtung nach einem der vorhergehenden Ansprüche, **dadurch gekennzeichnet, dass** sie extern ansteuerbar ist und Mittel zum Empfang von Steuerungs- und/oder Stromversorgungssignalen aufweist.

12. Vorrichtung nach einem der vorhergehenden Ansprüche, **dadurch gekennzeichnet, dass** zumindest Teile der Vorrichtung monolithisch und insbesondere mittels Mikroverfahrenstechnik, umfassend Mikrospritzgieß- oder Mikcoprägetechnik, hergestellt sind.

13. Vorrichtung nach einem der vorhergehenden Ansprüche, **dadurch gekennzeichnet, dass** sie im Inneren beschichtete Flächen zur Verhinderung des Anhaftens von Stoffen oder zur Katalyse von chemischen Reaktionen aufweist.

14. Vorrichtung nach einem der vorhergehenden Ansprüche, **dadurch gekennzeichnet, dass** sie eine Heiz- und/oder Kühlvorrichtung oder einen Teil davon umfasst.

15. Vorrichtung nach einem der vorhergehenden Ansprüche, **dadurch gekennzeichnet, dass** sie aus Kunststoff, insbesondere aus Polyethylen, Polypropylen, Polymethylmethacrylat, Cyclo-Olefin-Copolymer (COC), Polytetraflourethylen, Polycarbonat, Silizium, Metall oder Glas hergestellt ist.

16. System umfassend eine Vorrichtung nach einem der vorhergehenden Ansprüche und eine mit der Vorrichtung koppelbare Steuerungseinheit zur Steuerung der Vorrichtung.

17. System nach Anspruch 16, **dadurch gekennzeichnet, dass** es eine mit der Vorrichtung koppelbare Isotopenquelle und eine andere nicht in die Vorrichtung integrierte Quelle chemischer Substanzen umfasst.

18. System nach Anspruch 16 oder 17, **dadurch gekennzeichnet, dass** es ein mit der Vorrichtung koppelbares Förder- bzw. Dosiermittel, Mess- oder Sensormittel, ein Mittel zur Durchführung einer Chromatographie oder Elektrophorese, und/oder ein Mittel zur Erfassung der Lichtbrechung und/oder Erfassung zumindest einer Eigenschaft eines Stoffes aus der Gruppe umfassend das Vorhandensein oder die Abwesenheit, die Menge, die Farbe und den Brechungsindex, eine Ionenaustauschsäule, eine Größenausschlussextraktionssäule oder einen Teil davon umfasst.

19. Verfahren zur Herstellung einer geringen Menge einer radioaktiven Substanzverbindurig mit den Schritten:
- Bereitstellen einer zur Herstellung einer geringen Menge einer chemischen Substanzverbindung eingerichteten Vorrichtung gemäß einen der Ansprüche 1 bis 15;
- Zuführen einer geringen Menge zumindest einer Substanz in eine Mischvorrichtung (4) der Vorrichtung;
- Zuführen einer geringen Menge zumindest einer radioaktiven Substanz in die Mischvorrichtung (4);
- Mischen der zumindest einen Substanz mit der zumindest einen radioaktiven Substanz; und
- Entnehmen der hergestellten Substanzverbindung.

20. Verfahren nach Anspruch 19, **dadurch gekennzeichnet, dass** das Zuführen einer geringen Menge einer Substanz das Zuführen einer Menge von weniger als 2ml, bevorzugt von weniger als 1ml und besonders bevorzugt von weniger als 100µl umfasst.

21. Verfahren nach Anspruch 19 oder 20, **dadurch gekennzeichnet, dass** es das Zuführen einer Substanz aus einem in die Vorrichtung integrierten Behältnis umfasst.

22. Verfahren nach einem der Ansprüche 19 bis 21, **dadurch gekennzeichnet, dass** zumindest eine Substanz bei der Herstellung der Vorrichtung in ein Behältnis (3) der Vorrichtung eingebracht wird.

23. Verfahren nach einem der Ansprüche 19 bis 22, **dadurch gekennzeichnet, dass** es das Zuführen der radioaktiven Substanz von außerhalb der Vorrichtung umfasst.

24. Verfahren nach einem der Ansprüche 19 bis 23, **dadurch gekennzeichnet, dass** es das Kühlen oder Erwärmen von Substanzen umfasst.

25. Verfahren nach einem der Ansprüche 19 bis 24, **dadurch gekennzeichnet, dass** es das Durchführen einer Qualitätskontrolle einer oder mehrerer Substanzen oder Substanzverbindungen in der Vorrichtung umfasst.

26. Verfahren nach einem der Ansprüche 19 bis 25, **dadurch gekennzeichnet, dass** es das Durchführen einer Qualitätskontrolle der hergestellten radiochemischen Substanzverbindung vor einer Entnahme umfasst.

27. Verfahren nach Anspruch 25 oder 26, **dadurch gekennzeichnet, dass** die Qualitätskontrolle das Durchführen einer Größenausschlusschromatographie, einer Ionenaustauschchromatographie, und/oder einer Dünnschichtchromatographie umfasst.

28. Verfahren nach einem der Ansprüche 19 bis 27, **dadurch gekennzeichnet, dass** das Mischen das Radiomarkieren von Biomolekülen mit Isotopen umfasst.

29. Verfahren nach einem der Ansprüche 19 bis 28, **dadurch gekennzeichnet, dass** das Mischen eine chemische Bindung zwischen der radioaktiven Substanz und der zumindest einen Substanz zur Folge hat.

30. Verfahren nach einem der Ansprüche 19 bis 29, **dadurch gekennzeichnet, dass** die radioaktive Substanz aus der Gruppe umfassend Me²⁺, Me³⁺, MeO₄⁻ und Halogene gewählt ist.

31. Verfahren nach einem der Ansprüche 19 bis 30, **dadurch gekennzeichnet, dass** die radioaktive Substanz aus der Gruppe Cobalt-57, Cobalt-58, Selenium-75, Gallium-67, Gallium-68, Iodine-123, Iodine-124, Iodine-125, Iodine-131, Astatine-211, Actinium225, Bismuth-212, Bismuth-213, Lead-212, Technetium-99m, Rhenium-186, Rhenium-188, Silver-111, Indium-111, Platinum-197, Palladium-109, Copper-67, Phosphorus-32, Phosphorus-33, Yttrium-90, Scandium-47, Samarium-153, Ytterbium-169, Lutetium-177, Rhodium-105, Praseodymium-142, Praseodymium-143, Terbium-161, Holmium-166, Thallium-201or Gold-199 gewählt ist.

32. Verfahren nach einem der Ansprüche 19 bis 31, **dadurch gekennzeichnet, dass** es das Zuführen einer Pufferlösung gewählt aus der Gruppe umfassend Acetat, Zitrat, Phosphanate, Carbonate, HEPES, MES oder andere akzeptable Pufferlösungen umfasst.

33. Verfahren nach einem der Ansprüche 19 bis 32, **dadurch gekennzeichnet, dass** es das Steuern und Überwachen des Verfahrensablaufs mittels einer mit der Vorrichtung, koppelbaren Steuerungseinheit umfasst.

## Claims

1. Device for producing a small quantity of a radioactive substance compound comprising:
- a single-component body (6),
- a mixing device (4) which is integrated into the body (6) and used to receive a small quantity of chemical substances, and
- at least one container (3) which is integrated into the body (6) connected to the mixing device (4) and used to receive a small quantity of a chemical substance, **characterised in that**
- the mixing device (4) comprises a channel less than 500 µm high and less than 5 mm wide, so that a capillary effect is exerted on a liquid in the channel, and the channel comprises a rippled course between two straight sections, and
- the device comprises a mechanical interface (1, 2) to the outside and can be closed and sealed from the outside by means of the mechanical interface (1, 2).

2. Device according to claim 1 **characterised in that** it comprises one or more containers (3) integrated in the device and having a capacity of less than 5 ml, preferably of less than 100 µl and particularly preferably of less than 10 µl.

3. Device according to claim 1 or 2, **characterised in that** it comprises one or more channels (5) integrated in the device and having a capacity of less than 1 ml, preferably of less than 100 µl and particularly preferably of less than 10 µl.

4. Device according to claim 3, **characterised in that** the channel (5) is less than 100 µm high and preferably less than 25 µm high, and less than 500 µm wide and preferably less than 100 µm wide.

5. Device according to one of the claims 2 to 4, **characterised in that** the mixing device (4) and the at least one container (3) are connected by means of one or more channels (5).

6. Device according to one of the previous claims, **characterised in that** the mixing device (4) has an intake capacity of less than 1 ml, preferably less than 100 µl and particularly preferably less than 10 µl.

7. Device according to one of the previous claims, **characterised in that** the device can be closed and sealed from the outside.

8. Device according to one of the previous claims, **characterised in that** it comprises at least one access, in particular for a sensor.

9. Device according to one of the previous claims, **characterised in that** it comprises at least one conveying or dosing means from group comprising means working under the application of centrifugal force, an electrical force acting on a fluid, a change in pressure or volume or a sound-aided or vibration-aided conveying method, or a part thereof.

10. Device according to one of the previous claims, **characterised in that** it comprises at least one measuring or sensor means, in particular for recording a physical variable from the group comprising the type and strength of radioactive radiation, the pH value, the temperature, a means for carrying out chromatography or electrophoresis, and / or a means for recording the refraction and / or for recording at least one property of a substance from the group comprising the presence or the absence, the quantity, the colour and the refraction index, an ion exchange column, a size exclusion extraction column, or a part thereof.

11. Device according to one of the previous claims, **characterised in that** it can be controlled externally and comprises means to receive control and / or power supply signals.

12. Device according to one of the previous claims, **characterised in that** at least parts of the device are monolithic and are made in particular by means of micro process engineering, comprising micro injection moulding technology or micro embossing technology.

13. Device according to one of the previous claims, **characterised in that** it comprises internally coated surfaces to prevent the adhesion of substances or to catalyse chemical reactions.

14. Device according to one of the previous claims, **characterised in that** it comprises a heating and / or cooling device or a part thereof.

15. Device according to one of the previous claims, **characterised in that** it is made of plastic, in particular of polyethylene, polypropylene, polymethylmethacrylate, cyclo olefin copolymer (COC), polytetrafluorethylene, polycarbonate, silicon, metal or glass.

16. System comprising a device according to one of the previous claims and a control unit which can be coupled to the device, to control the device.

17. System according to claim 16, **characterised in that** it comprises an isotope source which can be coupled to the device and another source of chemical substances not integrated in the device.

18. System according to claim 16 or 17, **characterised in that** it comprises a conveying or dosing means which can be coupled to the device, measuring or sensor means, a means for carrying out chromatography or electrophoresis, and / or a means for recording the refraction and / or recording at least of one property of a substance from the group comprising the presence or the absence, the quantity, the colour and the refraction index, an ion exchange column, a size exclusion extraction column or a part thereof.

19. Method for producing a small quantity of a radioactive substance compound with the steps:
- Provision of a device used to produce a small quantity of a chemical substance compound according to one of the claims 1 to 15;
- Supply of a small quantity of at least one substance to a mixing device (4) of the device;
- Supply of a small quantity of at least one radioactive substance to the mixing device (4);
- Mixing of the at least one substance with the at least one radioactive substance; and
- Removal of the substance compound produced.

20. Method according to claim 19, **characterised in that** the supply of a small quantity of a substance comprises the supply of a quantity of less than 2 ml, preferably less than 1 ml and particularly preferably less than 100 µl.

21. Method according to claim 19 or 20, **characterised in that** it comprises the supply of a substance from a container integrated in the device.

22. Method according to one of the claims 19 to 21, **characterised in that** in the production of the device, at least one substance is introduced into a container (3) of the device.

23. Method according to one of the claims 19 to 22, **characterised in that** it comprises the supply of the radioactive substance from outside the device.

24. Method according to one of the claims 19 to 23, **characterised in that** it comprises the cooling or heating of substances.

25. Method according to one of the claims 19 to 24, **characterised in that** it comprises the carrying-out of a quality control of one or more substances or substance compounds in the device.

26. Method according to one of the claims 19 to 25, **characterised in that** it comprises the carrying-out of a quality control of the radio-chemical substance compound produced, before its removal.

27. Method according to claim 25 or 26, **characterised in that** the quality control comprises the carrying-out of variable exclusion chromatography, ion exchange chromatography, and / or thin-layer chromatography.

28. Method according to one of the claims 19 to 27, **characterised in that** the mixing comprises the radio-marking of biomolecules with isotopes.

29. Method according to one of the claims 19 to 28, **characterised in that** the mixing results in a chemical bond between the radioactive substance and the at least one substance.

30. Method according to one of the claims 19 to 29, **characterised in that** the radioactive substance is selected from the group comprising Me²⁺, Me³⁺, MeO₄ and halogens.

31. Method according to one of the claims 19 to 30, **characterised in that** the radioactive substance is selected from the group cobalt-57, cobalt-58, selenium-75, gallium-67, gallium-68, iodine-123, iodine-124, iodine-125, iodine-131, astatine-211, actinium-225, bismuth-212, bismuth-213, lead-212, technetium-99m, rhenium-186, rhenium-188, silver-111, indium-111, platinum-197, palladium-109, copper-67, phosphorus-32, phosphorus-33, yttrium-90, scandium-47, samarium-153, ytterbium-169, lutetium-177, rhodium-105, praseodymium-142, praseodymium-143, terbium-161, holmium-166, thallium-201 or gold-199.

32. Method according to one of the claims 19 to 31, **characterised in that** it comprises the supply of a buffer solution selected from the group comprising acetate, citrate, phosphanate, carbonate, HEPES, MES or other acceptable buffer solutions.

33. Method according to one of the claims 19 to 32, **characterised in that** it comprises the control and monitoring of the course of the method by means of a control unit which can be coupled to the device.

## Revendications

1. Dispositif de fabrication d'une faible quantité d'un composé de substances radioactif qui comprend :
- un corps (6) en une seule partie,
- un dispositif de mixage (4) intégré dans le corps (6) et aménagé pour la réception d'une faible quantité de substances chimiques, et
- au moins un récipient (3) intégré dans le corps (6) et raccordé au dispositif de mixage (4) et aménagé pour la réception d'une faible quantité d'une substance chimique, **caractérisé en ce que**
- le dispositif de mixage (4) comprend un canal d'une hauteur inférieure à 500 µm et d'une largeur inférieure à 5 mm de telle sorte qu'un effet de capillarité est exercé sur un liquide dans le canal, et de telle sorte que le canal présente un tracé ondulé entre deux tronçons rectilignes, et
- le dispositif présente une interface mécanique (1, 2) vers le côté extérieur et peut être fermé et rendu étanche vers l'extérieur au moyen de l'interface mécanique (1, 2).

2. Dispositif selon la revendication 1, **caractérisé en ce qu'**il présente un ou plusieurs récipients (3) intégrés dans le dispositif et contenant un volume inférieur à 5 ml, de préférence inférieur à 100 µl et de façon particulièrement préférée inférieur à 10 µl.

3. Dispositif selon la revendication 1 ou 2, **caractérisé en ce qu'**il présente un ou plusieurs canaux (5) intégrés dans le dispositif et contenant un volume inférieur à 1 ml, de préférence inférieur à 100 µl et de façon particulièrement préférée inférieur à 10 µl.

4. Dispositif selon la revendication 3, **caractérisé en ce que** le canal (5) présente une hauteur inférieure à 100 µm et de préférence inférieure à 25 µm et une largeur inférieure à 500 µm et de préférence inférieure à 100 µm.

5. Dispositif selon une des revendications 2 à 4, **caractérisé en ce que** le dispositif de mixage (4) et le récipient (3) au moins au nombre de un sont raccordés par un ou plusieurs canaux (5).

6. Dispositif selon une des revendications précédentes, **caractérisé en ce que** le dispositif de mixage (4) présente une capacité de réception inférieure à 1 ml, de préférence inférieure à 100 µl et de façon particulièrement préférée inférieure à 10 µl.

7. Dispositif selon une des revendications précédentes, **caractérisé en ce que** le dispositif peut être fermé et rendu étanche vers l'extérieur.

8. Dispositif selon une des revendications précédentes, **caractérisé en ce qu'**il présente au moins un accès, en particulier pour un capteur.

9. Dispositif selon une des revendications précédentes, **caractérisé en ce qu'**il comprend au moins un moyen de transport ou respectivement de dosage issu du groupe comprenant des moyens qui fonctionnent avec l'action de la force centrifuge, d'une force électrique agissant sur un fluide, d'une variation de pression ou de volume ou d'un procédé de transport assisté par le son ou les oscillations, ou une partie de ces moyens.

10. Dispositif selon une des revendications précédentes, **caractérisé en ce qu'**il comprend au moins un moyen de mesure ou de capteur, en particulier pour la détection d'une grandeur physique issue du groupe comprenant le type et la force de rayonnement radioactif, le pH, la température, un moyen d'exécution d'une chromatographie ou d'une électrophorèse, et/ou un moyen de détection de la réfraction de la lumière et/ou de détection d'au moins une caractéristique d'une matière issue du groupe comprenant la présence ou l'absence, la quantité, la couleur et l'indice de réfraction, une colonne d'échange d'ions, une colonne d'extraction par exclusion de taille ou une partie de ces moyens.

11. Dispositif selon une des revendications précédentes, **caractérisé en ce qu'**il peut être piloté extérieurement et présente des moyens de réception de signaux de commande et/ou d'alimentation électrique.

12. Dispositif selon une des revendications précédentes, **caractérisé en ce qu'**au moins des parties du dispositif sont fabriquées de façon monolithique et en particulier au moyen de la technique de micro-procédé, comprenant la technique de micromoulage par injection ou de microstructuration.

13. Dispositif selon une des revendications précédentes, **caractérisé en ce qu'**il présente à l'intérieur des surfaces revêtues destinées à empêcher l'adhérence de matières ou destinées à la catalyse de réactions chimiques.

14. Dispositif selon une des revendications précédentes, **caractérisé en ce qu'**il comprend un dispositif de chauffage et/ou de refroidissement ou une partie de celui-ci.

15. Dispositif selon une des revendications précédentes, **caractérisé en ce qu'**il est réalisé en matière plastique, en particulier en polyéthylène, en polypropylène, en polyméthylméthacrylate, en copolymère cyclo-oléfine (COC), en polytétrafluoroéthylène, en polycarbonate, en silicium, en métal ou en verre.

16. Système comprenant un dispositif selon une des revendications précédentes et une unité de commande pouvant être couplée au dispositif pour la commande du dispositif.

17. Système selon la revendication 16, **caractérisé en ce qu'**il comprend une source d'isotopes pouvant être couplée au dispositif et une autre source de substances chimiques non intégrée dans le dispositif.

18. Système selon la revendication 16 ou 17, **caractérisé en ce qu'**il comprend un moyen de transport ou respectivement de dosage pouvant être couplé au dispositif, des moyens de mesure ou de capteur, un moyen d'exécution d'une chromatographie ou d'une électrophorèse, et/ou un moyen de détection de la réfraction de la lumière et/ou de détection d'au moins une caractéristique d'une matière issue du groupe comprenant la présence ou l'absence, la quantité, la couleur et l'indice de réfraction, une colonne d'échange d'ions, une colonne d'extraction par exclusion de taille ou une partie de ces moyens.

19. Procédé de fabrication d'une faible quantité d'un composé de substances radioactif, comprenant les étapes suivantes :
- fourniture d'un dispositif de fabrication d'une faible quantité d'un composé de substances chimique selon une des revendications 1 à 15;
- acheminement d'une faible quantité d'au moins une substance vers un dispositif de mixage (4) du dispositif ;
- acheminement d'une faible quantité d'au moins une substance radioactive vers le dispositif de mixage (4) ;
- mixage de la substance au moins au nombre de un avec la substance radioactive au moins au nombre de un ; et
- enlèvement du composé de substances fabriqué.

20. Procédé selon la revendication 19, **caractérisé en ce que** l'acheminement d'une faible quantité d'une substance comprend l'acheminement d'une quantité inférieure à 2 ml, de préférence inférieure à 1 ml et de façon particulièrement préférée inférieure à 100 µl.

21. Procédé selon la revendication 19 ou 20, **caractérisé en ce qu'**il comprend l'acheminement d'une substance à partir d'un récipient intégré dans le dispositif.

22. Procédé selon une des revendications 19 à 21, **caractérisé en ce qu'**au moins une substance est mise en place dans un récipient (3) du dispositif lors de la fabrication du dispositif.

23. Procédé selon une des revendications 19 à 22, **caractérisé en ce qu'**il comprend l'acheminement de la substance radioactive à partir de l'extérieur du dispositif.

24. Procédé selon une des revendications 19 à 23, **caractérisé en ce qu'**il comprend le refroidissement ou le chauffage de substances.

25. Procédé selon une des revendications 19 à 24, **caractérisé en ce qu'**il comprend l'exécution d'un contrôle de qualité d'une ou de plusieurs substances ou de composés de substances dans le dispositif.

26. Procédé selon une des revendications 19 à 25, **caractérisé en ce qu'**il comprend l'exécution d'un contrôle de qualité du composé de substances radiochimique fabriqué avant un enlèvement.

27. Procédé selon la revendication 25 ou 26, **caractérisé en ce que** le contrôle de qualité comprend l'exécution d'une chromatographie d'exclusion de taille, d'une chromatographie par échange d'ions et/ou d'une chromatographie sur couche mince.

28. Procédé selon une des revendications 19 à 27, **caractérisé en ce que** le mixage comprend le radiomarquage de biomolécules avec des isotopes.

29. Procédé selon une des revendications 19 à 28, **caractérisé en ce que** le mixage a pour conséquence une liaison chimique entre la substance radioactive et la substance au moins au nombre de un.

30. Procédé selon une des revendications 19 à 29, **caractérisé en ce que** la substance radioactive est sélectionnée dans le groupe comprenant Me²⁺, Me³⁺, MeO₄⁻ et des halogènes.

31. Procédé selon une des revendications 19 à 30, **caractérisé en ce que** la substance radioactive est sélectionnée dans le groupe cobalt-57, cobalt-58, sélénium-75, gallium-67, gallium-68, iode-123, iode-124, iode-125, iode-131, astate-211, actinium-225, bismuth-212, bismuth-213, plomb-212, technétium-99m, rhénium-186, rhénium-188, argent-111, indium-111, platine-197, palladium-109, cuivre-67, phosphore-32, phosphore-33, yttrium-90, scandium-47, samarium-153, ytterbium-169, lutétium-177, rhodium-105, praséodymium-142, praséodymium-143, terbium-161, holmium-166, thallium-201 ou or-199.

32. Procédé selon une des revendications 19 à 31, **caractérisé en ce qu'**il comprend l'acheminement d'une solution tampon sélectionnée dans le groupe comprenant l'acétate, le citrate, le phosphanate, le carbonate, HEPES, MES ou d'autres solutions tampons acceptables.

33. Procédé selon une des revendications 19 à 32, **caractérisé en ce qu'**il comprend la commande et la surveillance du déroulement du procédé au moyen d'une unité de commande pouvant être couplée au dispositif.
